(19) Europäisches Patentamt
European Patent Office
Office européen des brevets

(11) Veröffentlichungsnummer: **0 441 218 A2**

# (12) EUROPÄISCHE PATENTANMELDUNG

(21) Anmeldenummer: **91101096.5**

(22) Anmeldetag: **28.01.91**

(51) Int. Cl.5: **C07H 15/252**, A61K 31/71, A61K 39/395

(30) Priorität: **01.02.90 DE 4002888**

(43) Veröffentlichungstag der Anmeldung: **14.08.91 Patentblatt 91/33**

(84) Benannte Vertragsstaaten: **AT BE CH DE DK ES FR GB GR IT LI LU NL SE**

(71) Anmelder: **BEHRINGWERKE Aktiengesellschaft**
**Postfach 1140**
**W-3550 Marburg 1(DE)**

(72) Erfinder: **Gerken, Manfred, Dr.**
**Wannkopfstrasse 12**
**W-3550 Marburg(DE)**
Erfinder: **Krause, Manfred**
**Bonameser Mittelgasse 2a**
**W-6000 Frankfurter am Main(DE)**
Erfinder: **Czech, Jörg, Dr.**
**Höhenweg 3**
**W-3550 Marburg(DE)**
Erfinder: **Bosslet, Klaus, Dr.**
**Am Schlag 5**
**W-3550 Marburg(DE)**
Erfinder: **Seemann, Gerhard, Dr.**
**Auf der Ebert 1**
**W-3550 Marburg(DE)**
Erfinder: **Hoffmann, Dieter, Dr.**
**Feuerdornweg 12**
**W-3550 Marburg(DE)**
Erfinder: **Sedlacek, Hans-Harald, Dr.**
**Sonnenhang 3**
**W-3550 Marburg(DE)**

(74) Vertreter: **Klein, Otto, Dr. et al**
**Hoechst AG Zentrale Patentabteilung**
**Postfach 80 03 20**
**W-6230 Frankfurt am Main 80(DE)**

## (54) Anthracyclin-Glycosyl-Prodrugs, Verfahren zu ihrer Herstellung und ihre Verwendung in Kombination mit funktionalisierten tumorspezifischen Enzymkonjugaten.

(57) Die vorliegende Erfindung bezieht sich nuf Glycosyl-Anthracyclin-Prodrugs, Verfahren zu ihrer Herstellung und ihrer Anwendung in Kombination mit funktionalisierten tumorspezifischen Enzym-Konjugaten für die Behandlung von Krebserkrankungen, und sie betrifft speziell 14-O-Glycosyl-Anthracycline als Prodrugs, die unter Einwirkung von tumorspezifischen Enzym-Konjugaten zu zytotoxischen Wirkstoffen gespalten werden können, wobei der freigesetzte Wirkstoff aufgrund seiner zytostatischen Wirksamkeit zur Behandlung von Krebserkrankungen geeignet ist.

Gegenstand der Erfindung sind 14-O-Glycosyl-Anthracycline der Formel I sowie deren Salze mit einer anorganischen oder organischen Säure

EP 0 441 218 A2

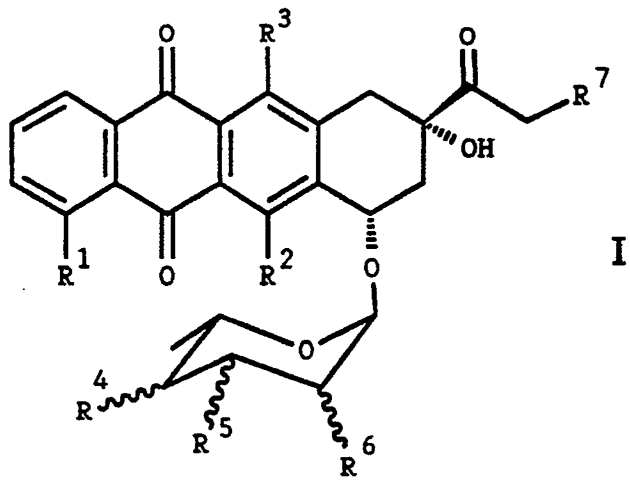

I

worin

$R^1$, $R^2$ und $R^3$

unabhängig voneinander Wasserstoff, Hydroxy, Methoxy,

$R^4$, $R^5$ und $R^6$

unabhängig voneinander Wasserstoff, Hydroxy, Halogen, aliphatisches Acyloxy (C1-C8), Benzoyloxy oder substituiertes Benzoyloxy wie para-Nitrobenzoyloxy, Alkyloxy (C1-C8), Allyloxy, Benzyloxy oder substituiertes Benzyloxy, Tetrahydropyranyloxy, Amino, NH-Acyl (C1-C8), NH-(9-Fluorenylmethoxycarbonyl), Morpholino oder substituiertes Morpholino, vorzugsweise 3-O-Methyl-morpholino oder 2-Cyano-morpholino,

$R^7$

ein Kohlenhydrat der allgemeinen Formel II

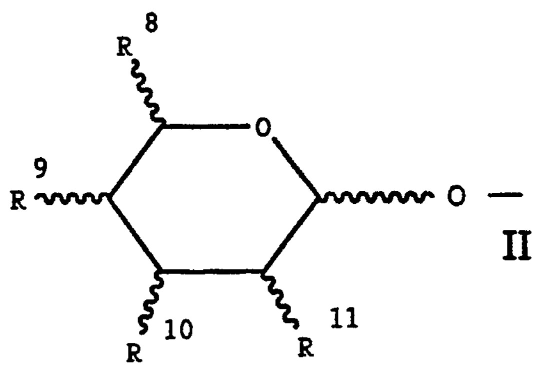

II

mit

$R^8$

Methyl, Hydroxymethyl, Acyloxymethyl (C1-C8), Alkyloxymethyl (C1-C8), Benzyloxymethyl, Allyloxymethyl, Carboxy, Carboxymethyl oder Carboxyallyl,

$R^9$, $R^{10}$ und $R^{11}$

unabhängig voneinander Wasserstoff, Hydroxy, Acyloxy (C1-C8), Benzoyloxy oder substituiertes Benzoyloxy wie para-Nitrobenzoyloxy, Alkyloxy (C1-C8), Allyloxy, Benzyloxy oder substituiertes Benzyloxy, Amino, NH-Acyl (C1-C8) oder NH-(9-Fluorenylmethoxycarbonyl) darstellen.

Ausgenommen werden Verbindungen, bei denen $R^4 = R^5 = R^9 = R^{10} =$ O-Acetyl und $R^6 = R^{11} = H$ in der Alpha-L-Desoxyfucose-Konformation

Unter einem funktionalisierten tumorspezifischen Enzym soll im Rahmen der Erfindung ein Enzym der Formel III

A-Sp-E III

verstanden werden, worin,

A

einen Antikörper oder eines seiner Fragmente, die Spezifität zu einem Tumor-assoziierten Antigen aufweisen,

oder ein in einem Tumor sich anreicherndes Biomolekül wie EGF (Epidermal Growth Factor), TGF-α - (Transforming Growth Factor α), PDGF (Platelet derived Growth Factor), IGF I+II (Insulin-like Growth Factor I+II) oder a+b FGF (acidic + basic Fibroblast Growth Factor)

E

eine nicht oder wenig immunogene Glycosidase, bevorzugt eine Säugetierglycosidase wie α- oder β-Glucosidase, α-Galactosidase, α- oder β-Mannosidase, α-Fucosidase, N-Acetyl-α-galactosaminidase, N-Acetyl-β-/N-Acetyl-α-glucosaminidase oder β-Glucuronidase, und

Sp

(Spacer) eine bifunktionelle Sulfid oder Disulfid enthaltende Gruppe der Formel IV

$X(S)_nY$ IV

oder $(S)_n$

worin

X oder Y

$-CO-R^{12}$-(N-succinimido)- oder $-C(=R^{13})-CH_2-CH_2-$ mit $R^{12}$ $-CH_2-CH_2-$, 1,4-Cyclohexyliden,1,3-oder 1,4-Phenylen oder Methoxycarbonyl-oder 1,4-Chlorophen-ylen und $R^{14}$ O oder NH,

Y

$-C(=R^{13})-CH_2-CH_2-$, wobei $R^{13}$ die angegebene Bedeutung hat, und

n

1 oder 2 bedeuten

oder einen Polypeptid-Spacer darstellt.

# ANTHRACYCLIN-GLYCOSYL-PRODRUGS, VERFAHREN ZU IHRER HERSTELLUNG UND IHRE VERWENDUNG IN KOMBINATION MIT FUNKTIONALISIERTEN TUMORSPEZIFISCHEN ENZYMKONJUGATEN

Die Erfindung betrifft Anthracyclin-Glycosyl-Prodrugs, Verfahren zu ihrer Herstellung und ihre Anwendung in Kombination mit funktionalisierten tumorspezifischen Enzym-Konjugaten für die Behandlung von Krebserkrankungen; dabei betrifft sie speziell 14-O-Glycosyl-Anthracycline als Prodrugs, welche durch Einwirkung von tumorspezifischen Enzymkonjugaten zu zytotoxischen Wirkstoffen gespalten werden können, wobei der freigesetzte Wirkstoff aufgrund seiner zytotoxischen Wirksamkeit zur Behandlung von Krebserkrankungen geeignet ist.

Ein Modell, in welchem Kombinationen von Prodrugs und tumorspezifischen Antikörper-Enzym-Konjugaten als therapeutische Mittel geprüft werden können, ist in der Fachliteratur beschrieben. Hierbei wurden gegen bestimmtes Gewebe gerichtete Antikörper, an die ein Prodrug-spaltendes Enzym kovalent gebunden ist, einem Tier, an welches dieses Gewebe transplantiert wurde, injiziert. Nach Lokalisation des MAK-Enzymkonjugates am Zielgewebe wird eine Enzym-aktivierbare Prodrug-Verbindung verabreicht. Unter der Einwirkung des am Gewebe verankerten Antikörper-Enzym-Konjugates wird das Prodrug zum Zellgift umgewandelt, das eine zytotoxische Wirkung gegen das transplantierte Gewebe ausübt.

In WO 88/07378 ist ein zwei Komponenten enthaltendes therapeutisches System, bestehend aus einer Antikörper-Enzym-Komponente und einer Enzym-aktivierbaren Prodrug-Komponente, beschrieben. Zur Herstellung der Antikörper-Enzym-Konjugate werden hierbei Nicht-Säuger-Enzyme verwendet und die Verwendung von endogenen Enzymen aufgrund möglicher unspezifischer Wirkstoff-Freisetzung ausgeschlossen. Da die exogenen Enzyme von dem Organismus als Fremdantigene erkannt werden, ist deren Verwendung mit dem Nachteil einer Immunantwort gegenüber diesen nicht körpereigenen Stoffen verbunden, aufgrund derer das am Antikörper immobilisierte Enzym desaktiviert und gegebenenfalls das ganze Konjugat eliminiert wird. Weiterhin werden hier p-Bis-N-(2-chlorethyl)-aminobenzyl-glutaminsäure und deren Derivate als Prodrug verwendet, deren chemische Halbwertzeit relativ kurz ist und vorzugsweise im Bereich von 5,0 bis 17,0 Stunden liegt. Die chemische Instabilität eines Prodrugs ist aufgrund der zu erwartenden Nebenwirkungen von Nachteil.

In EPA 0302473 A2 wird ebenfalls ein zwei Komponenten enthaltendes therapeutisches System beschrieben, in dem das am Tumorgewebe lokalisierte Antikörper-Enzym-Konjugat eine Prodrug-Verbindung zu einem zytotoxischen Wirkstoff spaltet.

Die hierbei unter anderem beschriebene kombinierte Verwendung eines N-Phenoxyacetylderivates von Adriamycin als Prodrug und Antikörper-immobilisierte L6-Penicillin V-Amidase zur Freisetzung von Adriamycin ist wegen der Immunogenität des bakteriellen Enzyms von Nachteil. Ein weiterer Nachteil solcher Prodrugs ist die durch die Acylierung der Aminogruppe des Adriamycins erhöhte Lipophilie der Prodrugs, wodurch die Löslichkeit im Plasma herabgesetzt wird und unspezifische Adsorption der Prodrugs an Zellmembranen stattfinden kann.

Die Synthese von 14-O-Glycosyl-Anthracyclinen ist bislang nur als ungewünschte Nebenreaktion der Glycosidierung von Adriamycinon mit 2-Desoxy-L-fucose beschrieben (Horton et al., Carbohydrate Research 94, 11-25, 1981).

Überraschenderweise zeigte sich, daß eine Glycosidierung von Anthracyclinen an der 14-Position, unter Berücksichtigung gewisser Schutzgruppenmuster am Anthracyclin, wie auch bei den Glycosyldonoren, möglich ist.

Ausgehend von dieser Erkenntnis sowie unter Berücksichtigung der oben beschriebenen Nachteile von bisherigen Kombinationen von Prodrugs und Antikörper-Enzym-Konjugaten hatte es sich die vorliegende Erfindung zur Aufgabe gemacht, synthetische, enzymatisch spaltbare 14-O-Glycosyl-Anthracycline sowie tumorspezifisch-funktionalisierte Enzyme,das heißt Enzyme, die in der Weise verändert sind, daß sie an Tumorzellen binden, die zur Therapie von Krebserkrankungen geeignet sind, herzustellen.

Gelöst wurde diese Aufgabe durch Bereitstellung von Verbindungen der Formel I sowie von tumorspezifisch-funktionalisierten Enzymen, die bei ihrer kombinierten Anwendung in der Prüfung auf zytostatische Aktivität eine Wirkung zeigten.

Gegenstand der Erfindung sind 14-O-Glycosyl-Anthracycline der Formel I sowie deren Salze mit einer anorganischen oder organischen Säure

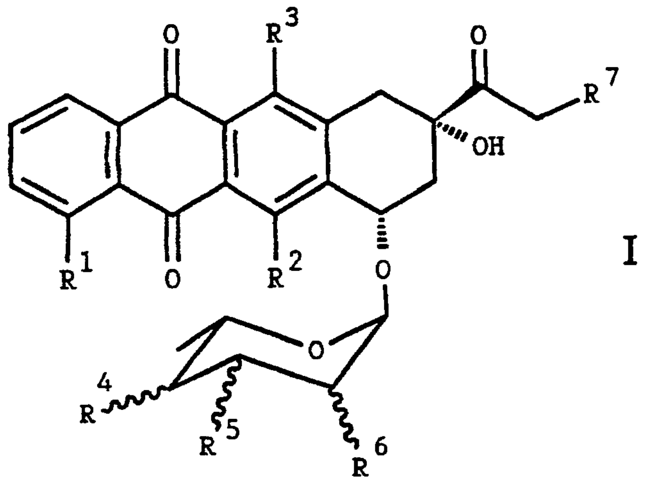

I

worin

$R^1$, $R^2$ und $R^3$

unabhängig voneinander Wasserstoff, Hydroxy, Methoxy,

$R^4$, $R^5$ und $R^6$

unabhängig voneinander Wasserstoff, Hydroxy, Halogen, aliphatisches Acyloxy (C1-C8), Benzoyloxy oder substituiertes Benzoyloxy wie para-Nitrobenzoyloxy, Alkyloxy (C1-C8), Allyloxy, Benzyloxy oder substituiertes Benzyloxy, Tetrahydropyranyloxy, Amino, NH-Acyl (C1-C8), NH-(9-Fluorenylmethoxycarbonyl), Morpholino oder substituiertes Morpholino, vorzugsweise 3-O-Methyl-morpholino oder 2-Cyano-morpholino,

$R^7$

ein Kohlenhydrat der allgemeinen Formel II

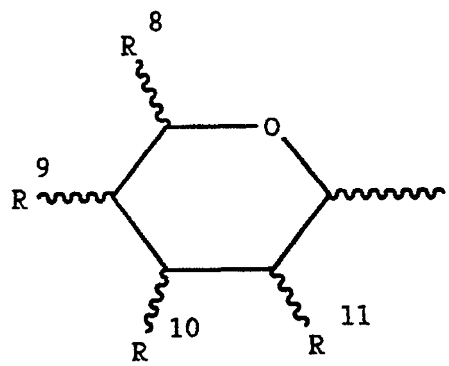

II

mit

$R^8$

Methyl, Hydroxymethyl, Acyloxymethyl (C1-C8), Alkyloxymethyl (C1-C8), Benzyloxymethyl, Allyloxymethyl, Carboxy, Carboxymethyl oder Carboxyallyl,

$R^9$, $R^{10}$ und $R^{11}$

unabhängig voneinander Wasserstoff, Hydroxy, Acyloxy (C1-C8), Benzoyloxy oder substituiertes Benzoyloxy wie para-Nitrobenzoyloxy, Alkyloxy (C1-C8), Allyloxy, Benzyloxy oder substituiertes Benzyloxy, Amino, NH-Acyl (C1-C8) oder NH-(9-Fluorenylmethoxycarbonyl) darstellen.

Ausgenommen werden Verbindungen, bei denen $R^4 = R^5 = R^9 = R^{10} =$ O-Acetyl und $R^6 = R^{11} = H$ in der Alpha-L-Desoxyfucose-Konformation sind.

Mit einer Acylgruppe ist auch eine Mono-, Di- oder Trihalogenacetylgruppe vorzugsweise mit den Halogenatomen Fluor oder Chlor gemeint.

Vorzugsweise ist mit substituiertem Benzyloxy ein mit einer oder zwei Methoxygruppen substituiertes Benzyloxy gemeint.

Unter einem funktionalisierten tumorspezifischen Enzym soll im Rahmen der Erfindung ein Enzym der Formel III

A-Sp-E III

verstanden werden, worin,

A

einen Antikörper oder eines seiner Fragmente, die Spezifität zu einem Tumor-assoziierten Antigen aufweisen, oder ein in einem Tumor sich anreicherndes Biomolekül wie EGF (Epidermal Growth Factor), TGF-α - (Transforming Growth Factor α), PDGF (Platelet derived Growth Factor), IGF I + II (Insulin-like Growth Factor I + II) oder a + b FGF (acidic + basic Fibroblast Growth Factor)

E

eine nicht oder wenig immunogene Glycosidase, bevorzugt eine Säugetierglycosidase wie α- oder ß-Glucosidase, α-Galactosidase, α- oder ß-Mannosidase, α-Fucosidase, N-Acetyl-α-galactosaminidase, N-Acetyl-ß-/N-Acetyl-α-glucosaminidase oder ß-Glucuronidase, und

SP

(Spacer) eine bifunktionelle Sulfid oder Disulfid enthaltende Gruppe der Formel IV

$X(S)_nY$ IV

oder $(S)_n$

worin

X oder Y

$-CO-R^{12}$-(N-succinimido)- oder $-C(=R^{13})-CH_2-CH_2-$ mit $R^{12}$ $-CH_2-CH_2-$, 1,4-Cyclohexyliden,1,3-oder 1,4-Phenylen oder Methoxycarbonyl-oder 1,4-Chlorophen-ylen und $R^{14}$ O oder NH,

Y

$-C(=R^{13})-CH_2-CH_2-$, wobei $R^{13}$ die angegebene Bedeutung hat, und

n

1 oder 2 bedeuten

oder einen Polypeptid-Spacer darstellt.

Zum Beispiel kann ein tumorspezifisch-funktionalisiertes Enzym, das ein Fusionsgen aus VH, CH1, Hinge und Enzymgen darstellt in ein Expressionsplasmid kloniert werden, das für die Expression in eukaryontischen Zellen geeignet ist und einen Selektionsmarker trägt. Das Expressionsplasmid mit dem Fusionsgen wird zusammen mit einem Expressionsplasmid, welches das zum Antikörper gehörende Leichte-Ketten-Gen enthält, in eukaryontische Expressionszellen transfiziert. Nach Selektion mit einem geeigneten Antibiotikum werden Transfektomaklone identifiziert, welche die Expressionsplasmide enthalten. Durch geeignete Nachweismethoden (BioDot, ELISA) werden solche Transfektomaklone identifiziert, die das Fusionsprotein der Formel III bestehend aus Antikörper und Enzym sezernieren.

Bevorzugt werden im Rahmen der Erfindung Verbindungen der Formel I, worin die Reste

$R^1$

Hydroxy oder Methoxy,

$R^2$ und $R^3$

Hydroxy,

$R^4$

Wasserstoff, Hydroxy, Tetrahydropyranyloxy, Amino oder Acyloxy (C1-C8),

$R^5$

Hydroxy, Acyloxy (C1-C8), Amino, NH-Acyl (C1-C8) oder NH-(9-Fluorenylmethoxycarbonyl),

$R^6$

Wasserstoff, Hydroxy, Halogen oder Acyloxy,

$R^7$

ein Kohlenhydrat der Formel II mit

$R^8$

Methyl, Hydroxymethyl, Acyloxymethyl (C1-C8), Alkyloxymethyl, Carboxy, Carboxymethyl, Carboxybenzyl oder Carboxyallyl, und

$R^9$, $R^{10}$, $R^{11}$

unabhängig voneinander Wasserstoff, Hydroxy, Acyloxy (C1-C8) oder Alkyloxy

darstellen, wobei die Verbindungen ausgenommen werden, bei denen $R^4 = R^5 = R^9 = R^{10} =$ 0-Acetyl und $R^6 = R^{11} = H$ in der α-L-Desoxyfucose-Konformation sind.

Weiterhin bevorzugt wird ein funktionalisiertes tumorspezifisches Enzym der Formel III, worin

A

ein Antikörper oder dessen Fragment, die Spezifität zu einem Tumor-assoziierten Antigen aufweisen, oder ein am oder im Tumor sich anreicherndes Biomolekül wie EGF (Epidermal Growth Factor), TGF-$\alpha$ - (Transforming Growth Factor $\alpha$), PDGF (Platelet derived Growth Factor), IGF I+II (Insulin like Growth Factor I+II) oder a+b FGF (acidic + basic Fibroblast Growth Factor)

E

eine nicht oder wenig immunogene Glycosidase, bevorzugt eine Säugetierglycosidase, besonders eine $\alpha$- oder $\beta$-Glucosidase, $\alpha$-Galactosidase, $\alpha$- oder $\beta$-Mannosidase, $\alpha$-Fucosidase, N-Acetyl-$\alpha$-galactosaminidase, N-Acetyl-$\beta$-/N-Acetyl-$\alpha$-glucosaminidase oder $\beta$-Glucuronidase,

Sp

eine bifunktionelle Disulfid enthaltende Gruppe der Formel IV oder $(S)_n$

$X\,(S)_n\,Y$ IV

darstellen, worin

X oder Y

-CO-$R^{12}$-(N-succinimido)- oder -C(=$R^{13}$)-$CH_2$-$CH_2$-mit $R^{12}$ -$CH_2$-$CH_2$- oder 1,4-Phenylen und $R^{13}$ O oder NH,

Y

-C(=$R^{13}$)-$CH_2$-$CH_2$-, wobei $R^{13}$ die angegebene Bedeutung hat, und

n

1 oder 2

bedeuten, oder Sp

einen geeigneten Polypeptid-Spacer darstellt.

Gegenstand der Erfindung ist auch ein Verfahren zur Herstellung einer Verbindung der Formel I mit den angegebenen Definitionen, dadurch gekennzeichnet, daß man ein Anthracyclin der Formel V

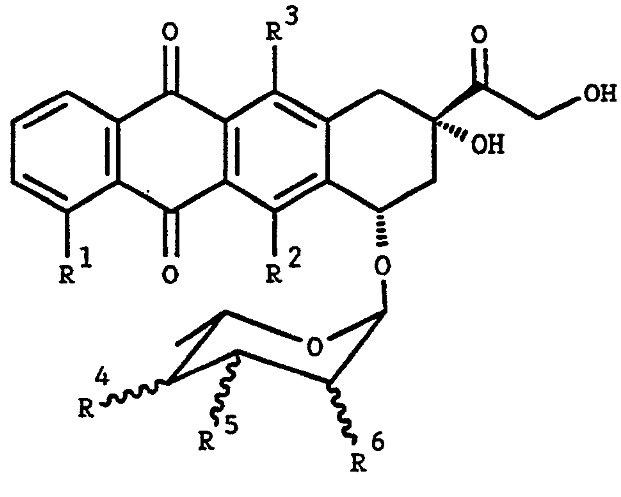

V

worin $R^1$, $R^2$, $R^3$, $R^4$, $R^5$ und $R^6$ die oben angegebenen Bedeutungen haben, mit einer Kohlenhydratkomponente der Formel VI

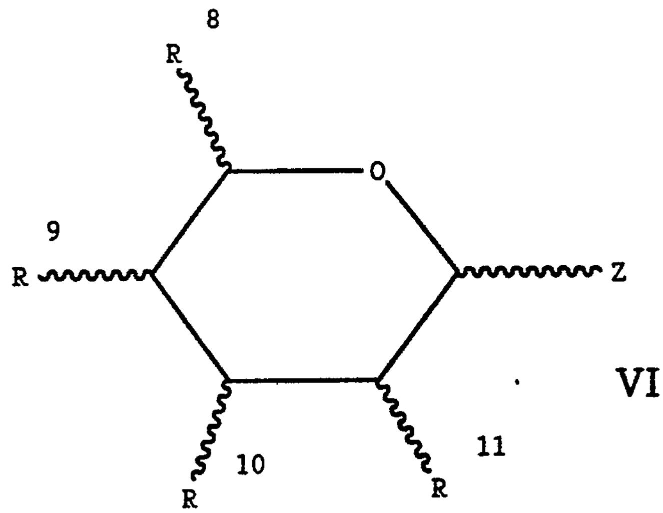

VI

worin

$R^8$

Methyl, Acyloxymethylen (C1-C8), Alkyloxymethyl (C1-C8), Benzyloxymethyl, Allyloxymethyl, Carboxymethyl, Carboxybenzyl oder Carboxyalkyl,

$R^9$, $R^{10}$, $R^{11}$

unabhängig voneinander Wasserstoff, Acyloxy (C1-C8), Benzoyloxy oder substituiertes Benzoyloxy wie para-Nitrobenzoyloxy, Alkyloxy (C1-C8), Allyloxy, Benzyloxy, substituiertes Benzyloxy, NH=Acyl oder NH-(9-Fluorenylmethoxycarbonyl) und

Z

eine Austrittsgruppe wie ein Halogenatom, eine Hydroxygruppe, eine Tri-C1-C4-alkylsilyloxy-Gruppe, eine über ein Sauerstoffatom gebundene Acyl-Schutzgruppe oder ein über ein Sauerstoffatom gebundenes Trichloracetimidat

darstellen,

in Gegenwart eines Promotors und gegebenenfalls eines Säurefängers und/oder Trockenmittels in einem Lösungsmittel bei -50° bis +80° C zu einem 14-O-Glycosyl-Anthracyclinderivat der Formel I, worin alle Reste $R^1$ bis $R^{11}$ ihre Bedeutung wie oben definiert beibehalten, umsetzt, und in diesen Verbindungen gegebenenfalls die vorhandenen Schutzgruppen oder einige der Schutzgruppen hydrolytisch oder mit Hilfe eines geeigneten Katalysators abspaltet.

Im einzelnen geht man dabei wie folgt vor:

Ein Anthracyclin der Formel V muß, wenn es als einen der Reste $R^4$, $R^5$ oder $R^6$ eine Aminofunktion trägt, zunächst an dieser Gruppe, zum Beispiel als NH-Trifluoracetat oder NH-(9-Fluorenylmethoxycarbonyl) geschützt werden.

Zur Glycosidierung an der 14-Position des Anthracyclins werden funktionalisierte Kohlenhydrat-Bausteine der Formel VI eingesetzt, die typischerweise als Reste $R^9$, $R^{10}$ und $R^{11}$ O-Acyl, O-Alkyl oder O-Allyl enthalten, wenn sie über Sauerstoff gebundene Reste tragen. Der Rest $R^8$ wird ebenfalls als Acyloxymethyl, Alkyloxymethyl oder Carboxyalkyl geschützt.

Am anomeren Zentrum müssen die Kohlenhydratderivate geeignete Austrittsgruppen Z haben. Glycosylhalogenide wie Fluoride, Chloride oder Bromide werden beispielsweise ausgehend von 1-O-Acyl-Derivaten mittels HF, HCl, HBr oder $TiBr_4$ hergestellt. Andere Verbindungen mit Austrittsgruppen wie Trichloracetimidate sowie mit verschiedenen Schutzgruppenmustern für $R^8$ bis $R^{11}$ werden nach den in der Kohlenhydratchemie üblichen Methoden hergestellt.

Zur Glycosidierung des kopplungsfähigen Anthracyclins mit einem Glycosyldonor ist ein Promotor nötig. Beim Einsatz von Glycosylfluoriden, 1-Hydroxy-Glycosiden,1-Acyloxy-Glycosiden oder 1-Trichloracetimidaten von Kohlenhydraten werden Lewissäuren wie $BF_3$xEther oderTrimethylsilyltriflat benutzt, während bei den Glycosylchloriden und -bromiden Silber- oder Quecksilbersalze verwendet werden.

Die Glycosidierung findet in einem aprotischen organischen Lösungsmittel oder Lösungsmittelgemisch wie Aceton, Ethylacetat, Ether, Toluol, Dichlormethan, Dichlorethan, Acetonitril, Nitromethan statt. Gegebenenfalls unter Zusatz eines Säurefängers oder Trockenmittels, z.B.eines Molekularsiebs, wird die Reaktion je nach Reaktivität des Glycosyldonors bei Temperaturen zwischen -50° und +80° C durchgeführt. Die anschließende Abspaltung der Schutzgruppen wird mit den in der Kohlenhydratchemie üblichen Methoden

durchgeführt.

Zur Herstellung von A-Sp-E kann entweder der Spacer (Sp) über eine Aminogruppe an ein Enzym und an den Antikörper oder das Biomolekül über eine eingeführte oder durch Spaltung einer Disulfidbrücke erzeugte HS-Gruppe verknüpft werden oder es werden Nukleinsäuresequenzen, die für die Teile A, Sp und E kodieren, mit Hilfe molekularbiologischer Methoden dergestalt kovalent verknüpft, daß ein Fusionsgen entsteht,und A-Sp-E mit gentechnischen Verfahren hergestellt.

Dies kann auf verschiedene Weisen erfolgen:

A) Eine Restriktionsschnittstelle A wird am 3' Ende des CH1 Exons im Gen der schweren Kette des Immunglobulins mit Hilfe spezifischer Mutagenese eingeführt. Die gleiche Restriktionsschnittstelle A wird am 5' Ende des Oligonukleotids generiert, welches für das Oligopeptid kodiert, das als Spacer fungiert. Beide Restriktionsschnittstellen A werden so gesetzt, daß das Immunglobulingen mit dem Oligonukleotid über die Restriktionsschnittstelle A verknüpft werden kann, ohne das Leseraster zu stören.

Am 3' Ende des Oligonukleotids wird eine Restriktionsschnittstelle B generiert. Diese Restriktionsschnittstelle B wird in dem Gen, das für das Enzym kodiert, an der Stelle eingeführt, an der die kodierende Nukleinsäuresequenz für das reife Protein beginnt. Dann wird das Enzymgen über die 'Restriktionsschnittstelle B mit dem Immunglobulingen-Linkerkonstrukt verknüpft. Die Restriktionsschnittstellen B werden so gesetzt, daß das Leseraster bei der Verknüpfung nicht gestört wird. Das Fusionsgen aus dem für die schweren Ketten des Immunglobulins VH und CH1-Linker-Enzym wird in ein Expressionsplasmid kloniert, das für die Expression in eukaryontischen Zellen geeignet ist und einen Selektionsmarker trägt.

Das Expressionsplasmid mit dem Fusionsgen wird zusammen mit einem weiteren Expressionsplasmid, welches das Gen für die zum Antikörper gehörende leichte Kette trägt, in eukaryontische Zellen transfiziert (z.B. Myelomzellen). Durch Selektion mit geeigneten Antibiotika werden Zellklone isoliert, welche die Plasmide mit dem Fusionsgen und dem Gen für die leichten Ketten enthalten (Transfektomas).

Durch geeignete Nachweismethoden (BioDot; ELISA)werden solche Transfektomas identifiziert, die das Fusionsprotein der Formel A-Sp-E bestehend aus MAk-Fab-Teil, Linkerpolypeptid und Enzym sezernieren.

B) Eine Restriktionsschnittstelle A wird am 3'-Ende des Hinge Exons des Gens für die schweren Ketten des Immunglobulins eingeführt. Die Restriktionsschnittstelle A wird an der Stelle des Enzymgens eingeführt, an der die kodierende Nukleotidsequenz für das reife Protein beginnt. Das Genfragment der schweren Ketten des Immunglobulins mit den VH-, CH1- und Hinge-Exons wird über die Restriktionsschnittstelle A mit dem Enzymgen verknüpft.

Die Restriktionsstellen A werden so gesetzt, daß bei der Verknüpfung das Leseraster nicht gestört wird. Der HingeTeil des Antikörpers hat in dieser Konstruktion die Funktion des Polypeptidspacers. Das Fusionsgen aus VH-, CH1-Hinge und Enzymgen wird in ein Expressionsplasmid kloniert, das für die Expression in eukaryontischen Zellen geeignet ist und einen Selektionsmarker trägt. Das Expressionsplasmid mit dem Fusionsgen wird zusammen mit einem weiteren Expressionsplasmid, welches das zum Antikörper gehörende Leichte-Ketten-Gen enthält, in eukaryontische Expressionszellen transfiziert. Nach Selektion mit einem geeigneten Antibiotikum werden Transfektomaklone identifiziert, die die Expressionsplasmide enthalten. Durch geeignete Nachweismethoden (BioDot, ELISA) werden solche Transfektomaklone identifiziert, die das Fusionsprotein der Formel III bestehend aus Antikörper und Enzym sezernieren.

Die Kopplung zwischen Enzym und Antikörper, dessen Fragment oder einem Biomolekül wird nach in der Literatur beschriebenen Verfahren durchgeführt (A. H. Blair und T. J. Ghose, J. Immunolog. Methods 59 (1983) 129-143; T.J. Ghose et al. Methods in Enzymology Vol. 93 (1983) 280-333). Hierbei wird zunächst das Enzym über dessen Aminogruppe mittels Succinimidyl-N-maleimido-alkyliden-, cycloalkyliden- oder arylen-1-carboxylat funktionalisiert, wobei die Doppelbindung der Maleimidofunktion in eine Reaktion mit der HS-Gruppe des funktionalisierten Antikörpers, dessen Fragmentes oder der Biomoleküle unter Herausbildung einer Thioetherfunktion eingeht. Zur Herstellung der Antikörper-Enzym-Konjugate können monoklonale Antikörper, beispielsweise die, die in EP-A-O 141 079 beschrieben sind, bevorzugt die Antikörper 431/26, 250/183, 704/152 und 494/32 verwendet werden. Die Spezifität dieser Antikörper gegenüber Tumorassoziierten Antigenen wurde bereits am Tier und Menschen mittels Immunszintigraphie und Immunhistochemie bewiesen. Die Nukleotidsequenz der V-Gene dieser monoklonalen Antikörper ist in der deutschen Patentanmeldung DE-A-39 09 799.4 beschrieben.

Zur Herstellung der tumorspezifischen Enzym-Konjugate können nachfolgend genannte Enzyme aus der bezeichneten Quelle gemäß der angegebenen Literaturvorschrift gereinigt werden:

- $\alpha$-Galactosidase A aus humaner Plazenta: Bishop, D.F. (1981), J. Biol. Chem. 256, 1307-1316, oder

aus transfizierten Säugetierzellen: Tsuji, S. (1987), Eur. J. Biochem. 165, 275-280.

- β-Glucuronidase aus humaner Plazenta: Brot, F.E.(1978) Biochemistry 17, 385-391
- humane β-Glucuronidase aus transfizierten Säugetierzellen: Oshima, A. (1987) Proc. Natl. Acad. Sci. USA 84, 685-689
- α-L-Fucosidase aus humaner Leber: Dawson, G., Tsay, G. (1977) Arch. Biochem. Biophys. 184, 12-23
- α-Mannosidase aus humaner Leber: Grabowski, G.A., Ikonne, J.U., Desnick, R.J. (1980) Enzyme 25, 13-25
- β-Mannosidase aus humaner Plazenta: Noeske, C., Mersmann, G. (1983) Hoppe Seylers Z Physiol. Chem. 364, 1645-1651
- α-Glucosidase aus humaner Magen-, Darmschleimhaut: Asp, N.-G., Gudmand-Hoeyer, E., Christiansen, P.M., Dahlquist, A. (1974) Scand. J. Clin. Lab Invest. 33, 15, 239-245
- β-Glucosidase aus humaner Leber: Daniels, L.B., Coyle, P.J., Chiao, Y.-B., Glew, R.H. (1981) J. Biol. Chem. 256, 13004-13013
- β-Glucocerebrosidase aus humaner Plazenta: Furbish, F.S., Blair, H.E., Shiloach, J., Pentcheu, P.G., Brady, R.O. (1977) Proc. Natl. Acad. Sci, USA 74, 3560-3563
- α-N-Acetylglucosaminidase aus humaner Plazenta: Roehrborn, W., von Figura, K. (1978) Hoppe Seylers Z. Physiol. Chem. 359, 1353-1362
- β-N-Acetylglucosaminidase aus humaner Amnionmembran: Orlacchio, A., Emiliani, C., Di Renzo, G.C., Cosmi, E.V. (1986) Clin. Chim. Acta 159, 279-289
- α-N-Acetylgalactosaminidase: gemäß Salvayre, R., Negre, A., Maret, A., Douste-Blazy, L. (1984) Pathol. Biol. (Paris) 32, 269-284.

Die glycolytische Aktivität der funktionalisierten tumorspezifischen Enzyme wurde in vergleichenden Untersuchungen mit p-Nitrophenylglycosiden beim jeweiligen pH-Optimum ermittelt.

Gegenstand der Erfindung ist weiterhin eine Verpackung enthaltend ein erfindungsgemäßes 14-O-Glycosyl-Anthracyclin und ein funktionalisiertes tumorspezifisches Enzym-Konjugat.

Zur Prüfung der Wirksamkeit einer kombinierten zeitlich versetzten Anwendung wurde Transplantat-Mäusen das funktionalisierte Enzym gegeben, gewartet bis zu dem Tag, an welchem der Plasmaspiegel des Enzyms praktisch auf Null gesunken war, das 14-O-Glycosyl-Anthracyclin gegeben und beobachtet, ob ein Wachstumsstop und eine Regression beim transplantierten Gewebe eintraten.

**Beispiele:**

Die folgenden Beispiele erläutern die Erfindung näher, ohne sie zu beschränken:

A Synthesen der Prodrugs:

Die Strukturen der hergestellten Verbindungen wurden mittels $^{1}H$- und $^{13}C$-NMR-Spektroskopie, unter Einbeziehung zweidimensionaler NMR-Methoden und anderer Multi-Puls-NMR-Techniken sowie MS-, UV- und IR-Spektroskopie ermittelt. Der Verlauf der Reaktionen sowie die resultierenden Verbindungen wurden dünnschichtchromatographisch oder mit der HPLC-Technik untersucht.

Beispiel 1:

2-O-Allyl-1,3,4,6-tetra-O-acetyl-α-D-galacto-hexo-pyranose (1)

Eine Lösung von 1,3,4,6-Tetra-O-acetyl-α-D-galactopyranose (3.48 g = 10 mmol) und Allyltrichloracetimidat (14 ml einer 30%igen Lösung in Hexan) in Dichlormethan (15 ml) wurde mit Trifluormethansulfonsäure (0.25 ml) versetzt und 4 h gerührt, wobei die Mischung auf 40 °C erwärmt wurde. Nach Abkühlen wurde filtriert, und das Filtrat mit wässriger Natriumhydrogencarbonatlösung ausgeschüttelt.

Die organische Phase wurde säulenchromatographisch getrennt (Laufmittel: Essigester/Petrolether = 1:4). Ausbeute: 1.52 g (3.9 mmol = 39 %), mp 133 °C, $[\alpha]_D^{25}$ + 68.6 (c = 1.0 in Dichlormethan).
$^{1}H$ NMR (300 MHz, $CDCl_3$) δ 6.40 (d, 1H, $J_{1,2}$ = 3.6 Hz, H-1), 5.83 (dddd, 1H, Allyl), 5.47 (dd, 1H, $J_{3,4}$ = 3.3 Hz, $J_{4,5}$ = 1.2 Hz, H-4), 5.32-5.17 (m, 3H, Allyl, H-3), 4.30 (dt, $J_{4,5}$ = 1.2 Hz, $J_{5,6a}$ = $J_{5,6b}$ = 6.6 Hz, H-5), 4.16-4.06 (m, 4H, H-6a, H-6b, Allyl), 3.88 (dd, 1H, $J_{1,2}$ = 3.6 Hz, $J_{2,3}$ = 10.5 Hz, H-2), 2.17 (s, 3H, OAc), 2.15 (s, 3H, OAc), 2.04 (s, 3H, OAc), 2.03 (s, 3H, OAc). $^{13}C$ NMR (50 MHz, $CDCl_3$) δ 117.4 (Allyl), 93.5 (Allyl), 89.7 (C-1), 71.8 (Allyl), 71.7 (C-2), 69.0 (C-3), 68.2 (C-5), 67.4 (C-4), 61.0 (C-6), 20.6 (OAc), 20.4 (OAc), 20.3 (OAc), 20.1 (OAc).

Beispiel 2:

2-O-Allyl-3,4,6-Tri-O-acetyl-α-D-galacto-hexo-pyranosyl bromid (2)

Verbindung 1 (940 mg = 2.42 mmol) wurde in Dichlormethan (2 ml) angelöst, bei 0° C mit HBr in Eisessig (11 ml; 33 % HBr Lösung) versetzt und zwei Stunden bei dieser Temperatur gerührt. Die Reaktionsmischung wurde auf Eis gegeben und mit Dichlormethan ausgeschüttelt. Die organische Phase wurde mit wässriger Natriumhydrogencarbonatlösung bis zur leicht basischen Reaktion ausgeschüttelt, über Natriumsulfat getrocknet und mehrfach mit Toluol abgezogen.

Ausbeute: 930 mg (2.27 mmol = 94 %), Sirup.

$^1H$ NMR (200 MHz, $CDCl_3$) δ 6.58 (d, 1H, $J_{1,2}$ = 3.8 Hz, H-1), 5.90 (dddd, 1H, Allyl), 5.49 (dd, 1H, $J_{3,4}$ = 3.4 Hz, $J_{4,5}$ = 1.3 Hz, H-4), 5.38-5.20 (m, 3H, H-3, Allyl), 4.48 (dt, 1H, $J_{4,5}$ = 1.3 Hz, $J_{5,6a}$ = $J_{5,6b}$ = 6.3 Hz, H-5), 4.23-4.04 (m, 4H, Allyl, H-6a, H-6b), 3.77 (dd, 1H, $J_{1,2}$ = 3.8 Hz, $J_{2,3}$ = 10.3 Hz, H-2), 2.17 (s, 3H, OAc), 2.08 (s, 3H, OAc), 2.03 (s, 3H, OAc).

$^{13}C$ NMR (50 MHz, $CDCl_3$) δ 170.2 (OAc), 169.7 (OAc), 169.6 (OAc), 133.7 (Allyl), 117.9 (Allyl), 90.6 (C-1), 72.8 (C-2), 71.7 (Allyl), 70.9 (C-5), 69.9 (C-3), 67.2 (C-4), 60.8 (C-6), 20.6 (OAc), 20.5 (OAc), 20.4 (OAc).

Beispiel 3:

N-(9-Fluorenylmethoxycarbonyl)-Doxorubicin (3)

Zu einer Suspension von Doxorubicin-Hydrochlorid (107 mg = 0.184 mmol) in absolutem Methanol (10 ml) wurde bis zur leicht alkalischen Reaktion Natriummethanolatlösung (30%ig in Methanol) gegeben, wobei sich die Substanz löste. Nach Zugabe von Natriumcarbonat (wasserfrei) (98 mg = 0.92 mmol) und 9-Fluorenylmethoxycarbonylchlorid (142 mg = 0.552 mmol) wurde eine Stunde bei Raumtemperatur gerührt. Nach Filtration und Einengen wurde säulenchromatographisch getrennt (Laufmittel:Dichlormethan/Methanol/Ameisensäure = 20:1:0.1).

Ausbeute: 139 mg (0.181 mmol = 98 %), mp 165 -166° C, $[\alpha]_D^{25}$ + 156° (c = 0.02 in Chloroform).

$^1H$ NMR (200 MHz, $CDCl_3$) δ 13.86 (s, 1H, OH-6), 13.09 (s, 1H, OH-11), 7.98-6.70 (m, 11H, H-1, H-2, H-3, 8 Fmoc-Aromaten H), 5.39 (bs, 1H, H-1'), 5.14 (m, 2H, NH, H-7), 4.71 (s, 2H, H-14), 4.48 (S, 2H, Fmoc-CH), 4.25 (s, 1H, Fmoc-CH), 4.07 (q, 1H, H-5'), 3.99 (s, 3H, OMe), 3.80 (m, 2H, H-3', H-4'), 3.55 (bs, 1H, OH), 3.15 (d, 1H, $J_{10a,10b}$ = 18.7 Hz, H-10a), 2.99 (bs, 1H, OH), 2.82 (d, 1H, $J_{10a,10b}$ = 18.7 Hz, H-10b), 2.48-2.03 (m, 4H, H-8a, H-8b, H-2a', H-2e'), 1.23 (d, 3H, $J_{5',6'}$ = 6.5 Hz, $CH_3$-6').

FAB-MS, m/z = 766 (M+H).

Beispiel 4:

14-O-(2-O-Allyl-3,4,6-tri-O-acetyl-α-D-galacto-hexo-pyranosyl)-N-trifluoracetyl-doxorubicin (4)

Zu einer Lösung von N-Trifluoroacetyl-doxorubicin (70 mg = 0.11 mmol) in einem Lösungsmittelgemisch aus absolutem Toluol und absolutem Nitromethan (7 ml; 1:1) mit Molekularsieb (4 Å) wurde Silbercarbonat (90 mg = 0.33 mmol) und Silberperchlorat (23 mg = 0.11 mmol) gegeben und eine Stunde bei Raumtemperatur gerührt. Zu diesem Gemisch wurde eine Lösung von Verbindung 2 (134 mg = 0.33 mmol) in absoluten Toluol (1 ml) gegeben und 22 Stunden bei Raumtemperatur gerührt. Nach Filtration und Nachwaschen mit Toluol und Zugabe von Dichlormethan wurde mit Natriumhydrogencarbonatlösung und dann mit Wasser ausgeschüttelt und die organische Phase über Natriumsulfat getrocknet. Durch Säulenchromatographie (Laufmittel Dichlormethan/ Methanol/Ameisensäure = 20:1:0.1) wurde das Produkt kristallin gewonnen.

Ausbeute: 48 mg (0.05 mmol = 45 %), mp 131-132° C, $[\alpha]_D^{25}$ + 220 (c = 0.02 in Chloroform).

$^1H$ NMR (300 MHz, $CDCl_3$) δ 14.01 (S, 1H, OH-6), 13.23 (s, 1H, OH-11), 8.04 (d, 1H, $J_{1,2}$ = 8.7 Hz, H-1), 7.79 (t, 1H, $J_{1,2}$ = $J_{2,3}$ = 8.5 Hz, H-2), 7.40 (d, 1H, $J_{2,3}$ = 8.3 Hz, H-3), 6.72 (d, 1H, $J_{3,NH}$ = 8.2 Hz, NH'), 5.93 (dddd, 1H, Allyl), 5.54 (d, 1H, $J_{3'',4''}$ = 3.4 Hz, H-4''), 5.50 (d, 1H, $J_{1',2a'}$ = 2.9 Hz, H-1'), 5.44-5.17 (m, 4H, H-7, H-3', 2 x Allyl), 5.15 (d, 1H, $J_{1'',2''}$ = 3.6 Hz, H-1''), 5.00 (d, 1H, $J_{14a,14b}$ = 19.5 Hz, H-14a), 4.86 (d, 1H, $J_{14a,14b}$ = 19.5 Hz, H-14b), 4.49 (t, 1H, $J_{5'',6a''}$ = $J_{5'',6b''}$ = 6.5 Hz, H-5''), 4.18 (m, 6H, 2 x Allyl, H-3', H-5', H-6a'', H-6b''), 4.09 (s, 3H, OMe), 3.87 (dd, 1H, $J_{1'',2''}$ = 3.5 Hz, $J_{2'',3''}$ = 10.5 Hz, H-2''), 3.67 (d, 1H, $J_{3',4'}$ = 3.7 Hz, H-4'), 3.27 (d, 1H, $J_{10a,10b}$ = 19.0 Hz, H-10a), 2.98 (d, 1H, $J_{10a,10b}$ = 19.0 Hz, H-10b), 2.36-1.7 (m, 4H, H-8a, H-8b, H-2a', H-2e'), 2.16 (s, 3H, OAc), 2.07 (s, 3H, OAc), 2.04 (s, 3H, OAc), 1.33 (d, 3H, $J_{5',6'}$ = 6.5 Hz, $CH_3$-6). FAB-MS, m/z = 990 (M + Na).

Beispiel 5:

14-O-(2-O-Allyl-3,4,6-tri-O-acetyl-α-D-galacto-hexo-pyranosyl)-N-(9-Fluorenylmethoxycarbonyl)-doxorubicin (5)

Verbindung 3 (93 mg = 0.122 mmol) wurde unter den in Beispiel 4 genannten Bedingungen mit Silbercarbonat (111 mg = 0.366 mmol), Silberperchlorat (25 mg = 0.122 mmol) und Verbindung 2 (150 mg = 0.366 mmol) umgesetzt und aufgearbeitet.
Ausbeute: 65 mg (0.06 mmol = 49 %), mp 148-149 °C, $[\alpha]_D^{25}$ +170 ° (c = 0.02 in Chloroform).
$^1$H NMR (200 MHz, $CDCl_3$) 13.97 (s, 1H, OH-6), 13.20 (s, 1H, OH-11), 8.08-7.01 (m, 11H, H-1, H-2, H-3, 8 x Fmoc-Aromaten H), 5.95 (dddd, 1H, Allyl), 5.52 (d, 1H, $J_{3'',4''}$ = 3.0 Hz, H-4"), 5.45 (d, 1H, $J_{1',2a'}$ = 2.8 Hz, H-1'), 5.43-5.11 (m, 5H, 2 x Allyl, H-7, H-1", H-3"), 4.94 (m, 2H, H-14a, H-14b), 4.57-4.03 (m, 13H, OMe, 2 x Allyl, 3 x Fmoc, H-6a", H-6b", H-3', H-5', H-5"), 3.87 (dd, 1H, $J_{1'',2''}$ = 3.3 Hz, $J_{2'',3''}$ = 10.4 Hz, 2"-H), 3.67 (m, 1H, H-4'), 3.25 (d, 1H, $J_{14a,14b}$ = 19.0 Hz, H-14a), 2.93 (d, 1H, $J_{14a,14b}$ = 19.0 Hz, H-14b, 2.15 (s, 3H, OAc), 2.06 (s, 3H, OAc), 2.03 (s, 3H, OAc), 1.31 (d, 3H, $J_{5',6'}$ = 6.3 Hz, $CH_3$-6').
FAB-MS, m/z = 1116 (M + Na).

Beispiel 6:

14-O-(3,4,6-Tri-O-acetyl-α-D-galacto-hexo-pyranosyl)-N-(9-Fluorenylmethoxycarbonyl)-doxorubicin (6)
Eine Lösung aus 1,5-Cyclooctadiene-bis[methyldiphenylphosphine]-iridium Hexafluorophosphat (5 mg) in absolutem Tetrahydrofuran (5 ml) wurde bis zur Entfärbung bei Atmosphärendruck hydriert und mit einer Lösung von Verbindung 5 (44 mg = 0.04 mmol) in absolutem Tetrahydrofuran versetzt und zwei Stunden gerührt. Anschließend wurde Wasser (1 ml) und Jod zugegeben und weitere 30 Minuten gerührt. Das Reaktionsgemisch wurde mit Dichlormethan versetzt und zunächst mit wässriger Natriumthiosulfatlösung, dann mit wässriger Natriumhydrogencarbonatlösung ausgeschüttelt. Nach Trocknen mit Natriumsulfat und Einengen wurde säulenchromatographisch (Laufmittel: Dichlormethan/Methanol/Ameisensäure = 20:1:0.1) gereinigt. Ausbeute: 15 mg (0.014 mmol = 35 %).

Beispiel 7:

14-O-(3,4,6-Tri-O-acetyl-α-D-galacto-hexo-pyranosyl)-doxorubicin (7)
Eine Lösung von Verbindung 6 (12 mg = 0.011 mmol) in Morpholin (2 ml) wurde drei Stunden bei Raumtemperatur gerührt und anschließend zweimal mit Toluol abgezogen. Nach Aufnehmen mit Dichlormethan wurde mit wässrigem Acetatpuffer (pH 5) ausgeschüttelt.
Ausbeute: 9 mg (0.0103 mmol = 94 %).
$^1$H NMR (200 MHz, $CDCl_3$) δ 8.03 (d, 1H, $J_{1,2}$ = 7.7 Hz, H-1), 7.79 (t, 1H, $J_{1,2}$ = $J_{2,3}$ = 8.0 Hz, H-2), 7.39 (d, 1H, $J_{2,3}$ = 8.2 Hz, H-3), 5.52 (m, 1H, H-4"), 5.46 (d, 1H, $J_{1',2a'}$ = 3.3 Hz, H-1'), 5.34 (m, 1H, H-7), 5.26 (dd, 1H, $J_{2'',3''}$ = 10.5 Hz, $J_{3'',4''}$ = 3.2 Hz, H-3"), 4.98 (d, 1H, $J_{1'',2''}$ = 3.8 Hz, H-1"), 4.92 (m, 2H, H-14a, H-14b), 4.39 (t, 1H, $J_{5'',6a''}$ = $J_{5'',6b''}$ = 6.5 Hz, H-5"), 4.14-3.97 (m, 6H, OMe, H-5', H-6a", H-6b"), 3.49 (s, 1H, H-4'), 3.27 (d, 1H, $J_{10a,10b}$ = 19.0 Hz, H-10a), 2.99 (d, 1H, $J_{10a,10b}$ = 19.0 Hz, H-10b), 2.15 (s, 3H, OAc), 2.07 (s, 6H, 2 x OAc), 1.36 (d, 3H, $J_{5',6'}$ = 6.6 Hz, $CH_3$-6).

Beispiel 8:

14-O-(3,4,6-Tri-O-acetyl-α-D-galacto-hexo-pyranosyl)-N-trifluoracetyl-doxorubicin (8)
Verbindung 4 wurde unter den bei Beispiel 7 genannten Bedingungen umgesetzt.
$^1$H NMR (200 MHz, $CDCl_3$) δ 14.05 (s, 1H, OH-6), 13.28 (s, 1H, OH-11), 8.06 (dd, 1H, $J_{1,2}$ = 7.8 Hz, $J_{1,3}$ = 0.8 Hz, H-1), 7.80 (t, 1H, $J_{1,2}$ = $J_{2,3}$ = 8.0 Hz, H-2), 7.40 (dd, 1H, $J_{2,3}$ = 8.7 Hz, $J_{1,3}$ = 0.8 Hz, H-3), 6.67 (d, 1H, $J_{3',NH}$ = 8.5 Hz, NH), 5.57 (d, 1H, $J_{3'',4''}$ = 2.8 Hz, H-4"), 5.45 (d, 1H, $J_{1',2a'}$ = 3.6 Hz, H-1'), 5.33 (m, 1H, H-7), 5.23 (dd, 1H, $J_{2'',3''}$ = 10.5 Hz, $J_{3'',4''}$ = 2.9 Hz, H-3"), 5.02 (d, 1H, $J_{1'',2''}$ = 3.8 Hz, H-1"), 4.87 (m, 2H, H-14a, H-14b), 4.37 (t, 1H, $J_{5'',6a''}$ = $J_{5'',6b''}$ = 6.5 Hz, H-5"), 4.20 (q, 1H, $J_{5',6'}$ = 6,3 Hz, H-5'), 4.09 (s, 3H, OMe), 3.65 (m, 1H, H-4'), 3.33 (d, 1H, $J_{10a,10b}$ = 18.5 Hz, H-10a), 2.98 (d, 1H, $J_{10a,10b}$ = 18.5 Hz, H-10b), 2.15 (s, 3H, OAc), 2.08 (s, 3H, OAc), 2.04 (s, 3H, OAc), 1.32 (d, 3H, $J_{5',6'}$ = 6.5 Hz, $CH_3$-6').

Beispiel 9:

14-O-(2-O-Allyl-α-D-galacto-hexo-pyranosyl)-N-trifluoroacetyl-doxorubicin (9)
Eine Lösung aus Verbindung 4 (15 mg = 0.015 mmol) in Methanol (2 ml) wurde mit einer katalytischen Menge Natriummethanolat versetzt und eine Stunde bei Raumtemperatur gerührt. Mit saurem Ionenaustauscher (Dowex WX50) wurde anschließend neutralisiert und nach Filtration eingeengt.
Ausbeute 12 mg (0.014 mmol = 93 %)
$^1$H NMR (400 MHz, $CDCl_3$ + $CD_3OD$) δ 14.02 (s, 1H, OH-6), 13.28 (s, 1H, OH-11), 8.03 (d, 1H, $J_{1,2}$ = 7.8

Hz, H-1), 7.80 (t, 1H, $J_{1,2}$ = $J_{2,3}$ = 8.0 Hz, H-2), 7.40 (d, 1H, $J_{2,3}$ = 8.5 Hz, H-3), 6.01 (m, 1H, Allyl), 5.55 (m, 1H, H-1'), 5.26 (m, 1H, H-7), 5.17 (d, 1H, $J_{1'',2''}$ = 3.5 Hz, H-1''), 4.94 (d, 1H, $J_{14a,14b}$ = 19.0 Hz, H-14a), 4.85 (d, 1H, $J_{14a,14b}$ = 19.0 Hz, H-14b), 4.08 (s, 3H, OMe), 3.28 (d, 1H, $J_{10a,10b}$ = 18.5 Hz, H-10a), 3.02 (d, 1H, $J_{10a,10b}$ = 18.5 Hz, H-10b), 1.32 (d, 3H, $J_{5',6'}$ = 6.5 Hz, $CH_3$-6').

Beispiel 10:

14-O-α-D-Galactohexopyranosyl-doxorubicin (10)

a) Verbindung 7 (4 mg = 0.005 mmol) wurde unter den bei Beispiel 9 genannten Bedingungen umgesetzt. Ausbeute: 2.8 mg (0.004 mmol = 80 %).

b) Eine Lösung von Verbindung 8 (11 mg = 0.012 mmol) in Methanol (1 ml) und 0.5 N Natronlauge (0.5 ml) wurde 90 Minuten bei Raumtemperatur gerührt. Nach Neutralisation mit verdünnter Salzsäure wurde mehrfach mit Toluol abgezogen, dann mit Methanol aufgenommen, über Celite filtriert und eingeengt. Die Reinigung erfolgte über HPLC (Laufmittel: Phosphatpuffer/Acetonitril/ Tetrahydrofuran = 30:60:10). $^1H$ NMR (200 MHz, $CDCl_3$ + $CD_3OD$) δ 7.90 (d, 1H, $J_{1,2}$ = 7.6 Hz, H-1), 7.73 (t, 1H, $J_{1,2}$ = $J_{2,3}$ = 8 Hz, H-2), 7.22 (d, 1H, $J_{2,3}$ = 8.4 Hz, H-3), 5.37 (s, 1H, H-1'), 5.12 (s, 1H,H-7), 4.85 (s, 2H, H-14a, H-14b), 4.75 (m, 1H, H-1''), 3.98 (s, 3H, OMe), 2.29 (d, 1H, $J_{10a,10b}$ = 15.5 Hz, H-10a), 2.06 (d, 1H, $J_{10a,10b}$ = 15.5 Hz, H-10b), 1.21 (d,3H, $J_{4',5'}$ = 6.6 Hz, $CH_3$-6).

B Spaltung der Prodrugs durch Enzyme:

Beispiel 11:

Spaltung von 14-O-α-D-Galacto-hexo-pyranosyl-doxorubicin (Verbindung 10) durch Human-Plazenta-α-Galactosidase A

0.5 mg der Ausgangsverbindung wurden in 0.5 ml Phosphat-Puffer, pH 5, enthaltend 0.03 U/ml der aus Human-Plazenta isolierten α-Galactosidase A gelöst und bei 37 °C im Dunklen inkubiert. Nach verschiedenen Inkubationszeiten wurden Proben des Inkubationsansatzes entnommen und direkt mittels Hochdruckflüssigkeitschromatographie analysiert (Säule: Nucleosil 100 (RP 18, 5 µm Teilchendurchmesser, 125 x 4.6 mm; Mobile Phase: Gradient aus Lösung A und B:
0 min - 20% A, 10 min - 30% A, 15 min - 30% A, Lösung A = 100% Acetonitril, Lösung B = 0.02 m Phosphat-Puffer pH 3.0; Flußrate: 1 ml/min; Detektion: Fluoreszenz, Ex 495 nm, Em 560 nm). Die Retentionszeit der Ausgangsverbindung betrug unter diesen Chromatographiebedingungen 9.0 min. Die während der Inkubation entstehende Verbindung wies eine mit Adriamycin identische Retentionszeit von 12.6 min auf.

Die Halbwertszeit der Spaltung betrug unter dem beschriebenen Bedingungen ca. 54 h (extrapolierter Wert). Bei der Inkubation der Ausgangsverbindung in enzymfreier Pufferlösung blieb die Verbindung über diesen Zeitraum stabil.

Beispiel 12:

Spaltung von 14-O-α-D-Galacto-hexo-pyranosyl-doxorubicin (Verbindung 10) durch Kaffebohnen-α-Galactosidase

Die Ausgangsverbindung wurde unter den gleichen wie in Beispiel 11 beschriebenen Bedingungen in Gegenwart von 0.03 U/ml Kaffeebohnen-α-Galactcsidase (Sigma) inkubiert. Die Halbwertszeit der Spaltung betrug in Gegenwart des Enzyms ca. 20 min.

C Bestimmung der Enzymaktivität von Enzym-Konjugaten:

Beispiel 13:

Bestimmung der Enzymaktivität von β-Glucuronidase-Konjugaten

Die nach oben genannter Vorschrift gereinigte β-Glucuronidase wurde an den Antikörper/das Biomolekül gekoppelt und die Aktivität des Enzyms sowie des Konjugates wie folgt bestimmt:

Zu 500 µl einer 2.5 mM p-Nitrophenyl-β-D-Glucuronidlösung in 100 mM HEPES (N-2-hydroxyethylpiperazine-N'-2-ethanesulfonic. acid), pH 5, wurden 500 µl der zu bestimmenden Enzymkonjugatlösung gegeben. Der Testansatz wurde bei 37° inkubiert und nach 6 min mit 500 µl einer 0.4 M Glycinlösung, pH 10.8, abgestoppt. Anschließend erfolgt die Bestimmung des freigesetzten p-Nitrophenols durch Messen der

Extinktion bei 405 nm.

Ergebnis:

Das Konjugat zeigte nur unwesentlich verringerte Enzymaktivität.

Beispiel 14:

Bestimmung der Enzymaktivität von α-D-Galactosidase-A-Konjugaten
Die nach oben genannter Vorschrift gereinigte α-Galactosidase A wurde an den Antikörper/das Biomolekül gekoppelt und die Aktivität des Enzyms sowie des Konjugates wie folgt bestimmt:

Zu 500 µl einer 2.5 mM p-Nitrophenyl-α-D-Galactopyranosidlösung in 100 mM HEPES (N-2-hydroxyethyl-piperazine-N'-2-ethane-sulfonic acid), pH 5, wurden 500 µl der zu bestimmenden Enzymkonjugatlösung gegeben. Der Testansatz wurde bei 37° inkubiert und nach 6 min mit 500 µl einer 0.4 M Glycinlösung, pH 10.8, abgestoppt. Anschließend erfolgt die Bestimmung des freigesetzten p-Nitrophenols durch Messen der Extinktion bei 405 nm.

Ergebnis:

Das Konjugat zeigte nur unwesentlich verringerte Enzymaktivität.

D Vorkommen der Enzyme in humanem Plasma:

Beispiel 15:

Vorkommen von β-D-Glucuronidase in humanem Plasma
Zu 500 µl einer 2.5 mM p-Nitrophenyl-β-D-Glucuronidlösung in 100 mH HEPES (N-2-hydroxyethyl-piperazine-N'-2-ethanesulfonic acid), pH 7.4, wurden 500 µl humanes Plasma gegeben. Der Testansatz wurde bei 30° inkubiert und nach 30 min mit 500 µl einer 0.4 M Glycinlösung, pH 10.8, abgestoppt. Anschließend erfolgt die Bestimmung des freigesetzten p-Nitrophenols durch Messen der Extinktion bei 405 nm.

Ergebnis:

Unter den Versuchsbedingungen konnte keine Enzymaktivität für β-D-Glucuronidase festgestellt werden.

Beispiel 16:

Vorkommen von α-D-Galactosidase in humanem Plasma
Zu 500 µl einer 2.5 mM p-Nitrophenyl-α-D-Galactopyranosidlösung in 100 mM HEPES (N-2-hydroxyethyl-piperazine-N'-2-ethane-sulfonic acid), pH 7.4, wurden 500 µl humanes Plasma gegeben. Der Testansatz wurde bei 30° inkubiert und nach 30 min mit 500 µl einer 0.4 M Glycinlösung, pH 10.8, abgestoppt. Anschließend erfolgt die Bestimmung des freigesetzten p-Nitrophenols durch Messen der Extinktion bei 405 nm.

Ergebnis:

Unter den Versuchsbedingungen konnte keine Enzymaktivität für α-D-Galactosidase festgestellt werden.

E In vivo anti Tumoreffekte:

Beispiel 17:

In vivo anti Tumoreffekte des 14-O-Glycosyl-Anthracyclin-Prodrug Systems
30 NMRI nu/nu Mäuse erhielten an Tag 0 pro Tier eine subkutane Inokulation von ca. 5 mm großen CoCa 4 Humantumorstückchen. Nach Anwachsen der Humantumorgewebe in den Mäusen (Tag 7-14) erhielten je 6 Tiere der Gruppen

a,b,c 5x 500 µg MAk BW 494/32-Galactosidase Konjugat,
d 5 x 500 µg MAk BW 494/32,
e 5 x 500 µg Galactosidase
und 5 x 500 µl PBS an 5 aufeinanderfolgenden Tagen intravenös injiziert.

Am 5., 6. und 7. Tag nach Beendigung der MAk BW 494/32-Galactosidase-, MAk BW 494/32-, Galactosidase- oder PBS-Injektion erhielten die Mäuse der Gruppen a, d und e pro Tier ein Drittel der maximal tolerablen Dosis (MTD) des 14-O-Glycosyl-Anthracyclins pro Tag intravenös injiziert. Die Mäuse der Gruppe b erhielten je 1/10 der MTD, die der Gruppe c 1/20 der MTD an den gleichen Tagen.

Ergebnis:

In den Gruppen d und e zeigte sich ein Tumorwachstum, welches sich nicht signifikant von dem der Gruppe f unterschied. Die Gruppen a, b und c zeigten eine deutliche Tumorwachstumsinhibition, wobei die Effekte in der Gruppe a am deutlichsten waren.

Vergleichbare Ergebnisse wurden mit den MAk BW 431/26, BW 250/183 im CoCa4 Xenograftsystem sowie mit dem MAk BW 704 im M21 Xenograftsystem erhalten.

**Patentansprüche**

1. Verbindungen der Formel I sowie deren Salze mit einer anorganischen oder organischen Säure

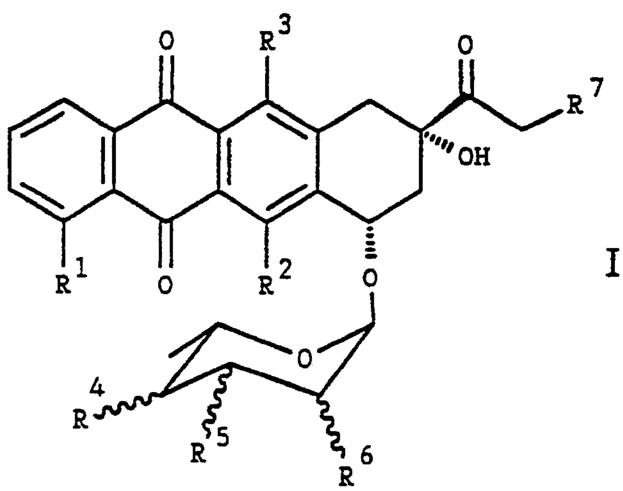

I

worin
$R^1$, $R^2$ und $R^3$
unabhängig voneinander Wasserstoff, Hydroxy, Methoxy,
$R^4$, $R^5$ und $R^6$
unabhängig voneinander Wasserstoff, Hydroxy, Halogen, aliphatisches Acyloxy (C1-C8), Benzoyloxy oder substituiertes Benzoyloxy wie para-Nitrobenzoyloxy, Alkyloxy (C1-C8), Allyloxy, Benzyloxy oder substituiertes Benzyloxy, Tetrahydropyranyloxy, Amino, NH-Acyl (C1-C8), NH-(9-Fluorenylmethoxycarbonyl), Morpholino oder substituiertes Morpholino, vorzugsweise 3-O-Methyl-morpholino oder 2-Cyanomorpholino,
$R^7$
ein Kohlenhydrat der Formel II

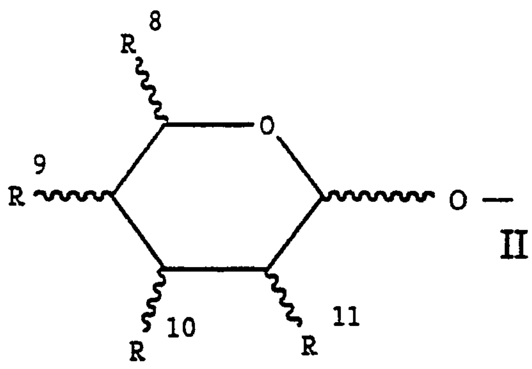

II

mit

$R^8$

Methyl, Hydroxymethyl, Acyloxymethyl (C1-C8), Alkyloxymethyl (C1-C8), Benzyloxymethyl, Allyloxymethyl, Carboxy, Carboxymethyl oder Carboxyallyl,

$R^9$, $R^{10}$ und $R^{11}$

unabhängig voneinander Wasserstoff, Hydroxy, Acyloxy (C1-C8), Benzoyloxy oder substituiertes Benzoyloxy wie para-Nitrobenzoyloxy, Alkyloxy (C1-C8), Allyloxy, Benzyloxy oder substituiertes Benzyloxy, Amino, NH Acyl (C1-C8) oder NH-(9-Fluorenylmethoxycarbonyl),

wobei eine Acylgruppe eine Mono-, Di- oder Trihalogenacetylgruppe vorzugsweise mit den Halogenatomen Fluor oder Chlor ist, darstellen, wobei Verbindungen, bei denen $R^4 = R^5 = R^9 = R^{10}$ = 0-Acetyl und $R^6 = R^{11}$ = H in der α-L-Desoxyfucose-Konformation ausgenommen sind.

2. Verbindung nach Anspruch 1, dadurch gekennzeichnet, daß $R^1$ Methoxy, $R^2$, $R^3$ Hydroxy und die Reste $R^4$ bis R $^{11}$ die in Anspruch 1 angegebene Bedeutung haben.

3. Verbindung nach Anspruch 1, dadurch gekennzeichnet, daß $R^1$ Methoxy, $R^2$ und $R^3$ Hydroxy und $R^4$ Hydroxy oder Tetrahydropyranyloxy, $R^5$ Amino, $R^6$ Wasserstoff und $R^7$ein Kohlenhydrat in der α-D-Galactopyranosyl- oder β-D-Glucuronyl-Konfiguration mit den in Anspruch 1 angegebenen Bedeutungen für $R^8$ bis $R^{11}$ sind.

4. Verbindung nach Anspruch 1, dadurch gekennzeichnet, daß $R^1$ Methoxy, $R^2$, $R^3$ Hydroxy und $R^4$ Hydroxy oder Tetrahydropyranyloxy, $R^5$ Amino, $R^6$ Wasserstoff, $R^7$ α-D-Galactopyranosyl mit $R^8$ Hydroxymethyl und $R^9$, $R^{10}$ und $R^{11}$ Hydroxy ist.

5. Verbindung nach Anspruch 1, dadurch gekennzeichnet, daß $R^1$ Methoxy, $R^2$, $R^3$ Hydroxy und $R^4$ Hydroxy oder Tetrahydropyranyloxy, $R^5$ Amino, $R^6$ Wasserstoff, $R^7$ β-D-Glucuronyl mit $R^8$ Carboxy und $R^9$, $R^{10}$ und $R^{11}$ Hydroxy ist.

6. Verbindung nach Anspruch 1, dadurch gekennzeichnet, daß $R^1$ Methoxy, $R^2$, $R^3$ und $R^4$ Hydroxy, $R^5$ Amino, $R^6$ Wasserstoff, $R^7$ α-D-Galactopyranosyl mit $R^8$ Hydroxymethyl und $R^9$, $R^{10}$ und $R^{11}$ Hydroxy ist.

7. Verbindung nach Anspruch 1, dadurch gekennzeichnet, daß $R^1$ Methoxy, $R^2$, $R^3$ und $R^4$ Hydroxy, $R^5$ Amino, $R^6$ Wasserstoff, $R^7$ β-D-Glucuronyl mit $R^8$ Carboxy und $R^9$, $R^{10}$ und $R^{11}$ Hydroxy ist.

8. Verfahren zur Herstellung einer Verbindung nach Anspruch 1 , dadurch gekennzeichnet, daß man ein Anthracyclin der Formel V

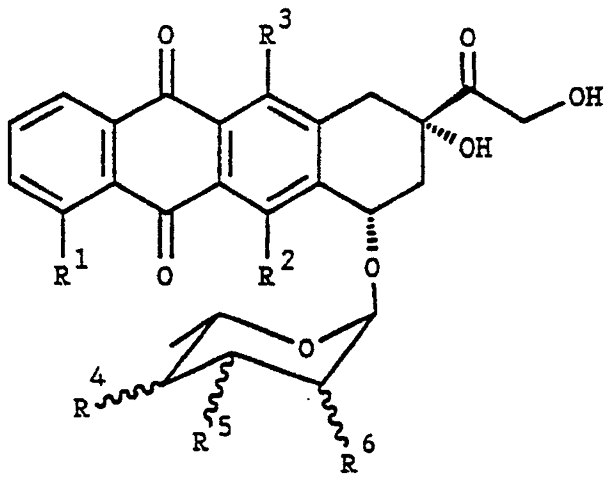

V

worin $R^1$, $R^2$, $R^3$, $R^4$, $R^5$ und $R^6$ die oben angegebenen Bedeutungen haben, mit einer Kohlenhydratkomponente der Formel VI

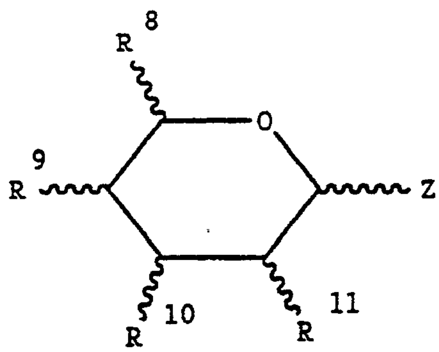

VI

worin

$R^8$

Methyl, Acyloxymethylen (C1-C8), Alkyloxymethyl (C1-C8), Benzyloxymethyl, Allyloxymethyl, Carboxymethyl, Carboxybenzyl oder Carboxyalkyl,

$R^9$, $R^{10}$ und $R^{11}$

unabhängig voneinander Wasserstoff, Acyloxy (C1-C8), Benzoyloxy oder substituiertes Benzoyloxy wie para-Nitrobenzoyloxy, Alkyloxy (C1-C8), Allyloxy, Benzyloxy, substituiertes Benzyloxy NH-Acyl oder NH-(9-Fluorenylmethoxycarbonyl) und

Z

eine Austrittsgruppe wie ein Halogenatom, eine Hydroxygruppe, eine Tri-C1-C4-alkylsilyloxy-Gruppe, eine über ein Sauerstoffatom gebundene Acyl-Schutzgruppe, oder ein über Sauerstoffatom gebundenes Trichloracetimidat

darstellen,

in Gegenwart eines Promotors und gegebenenfalls eines Säurefängers und/oder Trockenmittels in einem Lösungsmittel bei -50° bis +80° C zu einem 14-O-Glycosyl-Anthracyclinderivat der Formel I, worin alle Reste $R^1$ bis $R^{11}$ ihre Bedeutung wie oben definiert beibehalten, umsetzt, und in diesen Verbindungen gegebenenfalls die vorhandenen Schutzgruppen oder einige der Schutzgruppen hydrolytisch oder mit Hilfe eines geeigneten Katalysators abspaltet.

**9.** Verpackungseinheit enthaltend eine Verbindung nach mindestens Ansprüche 1 bis 7 und ein funktionalisiertes tumor- einem der spezifisches Enzym der Formel III

A-Sp-E III

worin

A
ein Antikörper oder dessen Fragment, die Spezifität zu einem Tumor-assoziierten Antigen aufweisen, oder ein am oder im Tumor sich anreicherndes Biomolekül wie EGF (Epidermal Growth Factor), TGF-$\alpha$ (Transforming Growth Factor $\alpha$), PDGF (Platelet derived Growth Factor), IGF I+II (Insulin like Growth Factor I+II) oder a+b FGF (acidic + basic Fibroblast Growth Factor),
E
eine nicht oder wenig immunogene Glycosidase, bevorzugt eine Säugetierglycosidase, besonders eine $\alpha$- oder $\beta$-Glucosidase, $\alpha$-Galactosidase, $\alpha$- oder $\beta$-Mannosidase, $\alpha$-Fucosidase, N-Acetyl-$\alpha$-galactosaminidase, N-Acetyl-$\beta$-/N-Acetyl-$\alpha$-glucosaminidase oder $\beta$-Glucuronidase,
Sp
eine bifunktionelle Disulfid enthaltende Gruppe der Formel IV

$X\,(S)_n\,Y$     IV

oder $(S)_n$
darstellen, worin
X oder Y
-CO-$R^{12}$-(N-succinimido)- oder -C(=$R^{13}$)-$CH_2$-$CH_2$-mit $R^{12}$ -$CH_2$-$CH_2$- oder 1,4-Phenylen und $R^{13}$ O oder NH,
Y
-C(=$R^{13}$)-$CH_2$-$CH_2$, wobei $R^{13}$ die angegebene Bedeutung hat, und
n
1 oder 2
oder einen Polypeptid-Spacer bedeuten.

**10.** Verpackungseinheit nach Anspruch 9, dadurch gekennzeichnet, daß A und E mittels Sp chemisch miteinander verbunden wurden.

**11.** Verpackungseinheit nach Anspruch 9, dadurch gekennzeichnet, daß A, Sp und E durch Kontruktion eines Fusionsgens miteinander verbunden wurden, wobei Sp einen Polypeptidspacer darstellt.

**12.** Verpackungseinheit nach Anspruch 11, dadurch gekennzeichnet, daß A-Sp-E von einer mit dem Fusionsgen transfizierten Wirtszelle produziert wird.

**13.** Verpackungseinheit nach Anspruch 11, dadurch gekennzeichnet, daß die Wirtszelle eukaryontischen Ursprungs ist.

**14.** Verpackungseinheit nach Anspruch 11, dadurch gekennzeichnet, daß die Wirtszelle prokaryontischen Ursprungs ist.

**15.** Arzneimittel enthaltend eine Verbindung nach mindestens einem der Ansprüche 1 bis 7.

**16.** Verwendung einer Verbindung nach mindestens einem der Ansprüche 1 bis 7 als Tumortherapeutikum.

**Patentansprüche für folgende Vertragsstaaten: ES,GR**

**1.** Verfahren zur Herstellung einer Verbindung der Formel I sowie deren Salze mit einer anorganischen oder organischen Säure

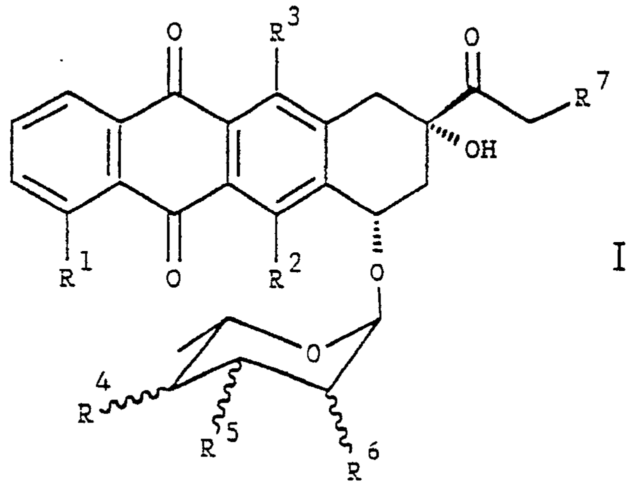

I

worin

$R^1$, $R^2$ und $R^3$

unabhängig voneinander Wasserstoff, Hydroxy, Methoxy,

$R^4$, $R^5$ und $R^6$

unabhängig voneinander Wasserstoff, Hydroxy, Halogen, aliphatisches Acyloxy (C1-C8), Benzoyloxy oder substituiertes Benzoyloxy wie para-Nitrobenzoyloxy, Alkyloxy (C1-C8), Allyloxy, Benzyloxy oder substituiertes Benzyloxy, Tetrahydropyranyloxy, Amino, NH Acyl (C1-C8), NH-(9-Fluorenylmethoxycarbonyl), Morpholino oder substituiertes Morpholino, vorzugsweise 3-O-Methyl-morpholino oder 2-Cyanomorpholino,

$R^7$

ein Kohlenhydrat der Formel II

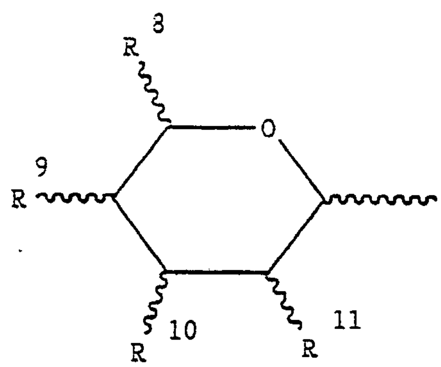

II

mit

$R^8$

Methyl, Hydroxymethyl, Acyloxymethyl (C1-C8), Alkyloxymethyl (C1-C8), Benzyloxymethyl, Allyloxymethyl, Carboxy, Carboxymethyl oder Carboxyallyl,

$R^9$, $R^{10}$ und $R^{11}$

unabhängig voneinander Wasserstoff, Hydroxy, Acyloxy (C1-C8), Benzoyloxy oder substituiertes Benzyloxy wie para-Nitrobenzoyloxy, Alkyloxy (C1-C8), Allyloxy, Benzyloxy oder substituiertes Benzyloxy, Amino, NH Acyl (C1-C8) oder NH-(9-Fluorenylmethoxycarbonyl),

wobei eine Acylgruppe eine Mono-, Di- oder Trihalogenacetylgruppe vorzugsweise mit den Halogenatomen Fluor oder Chlor ist, darstellen, wobei Verbindungen, bei denen $R^4 = R^5 = R^9 = R^{11} = 0$ Acetyl und $R^6 = R^{11} = H$ in der $\alpha$-L-Desoxyfucose-Konformation ausgenommen sind, dadurch gekennzeichnet, daß man ein Anthracyclin der Formel V

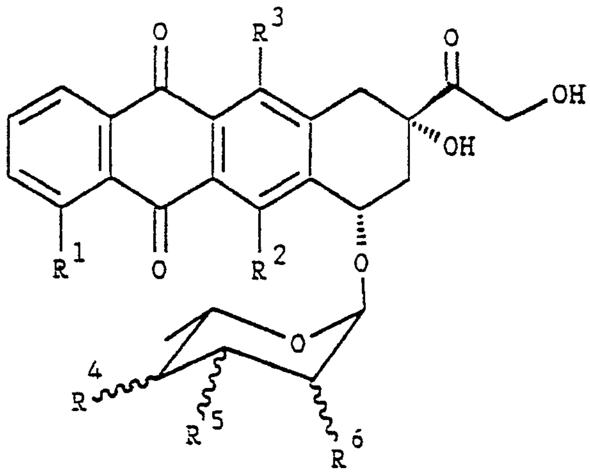

V

worin $R^1$, $R^2$, $R^3$, $R^4$, $R^5$ und $R^6$ die oben angegebenen Bedeutungen haben, mit einer Kohlenhydratkomponente der Formel VI

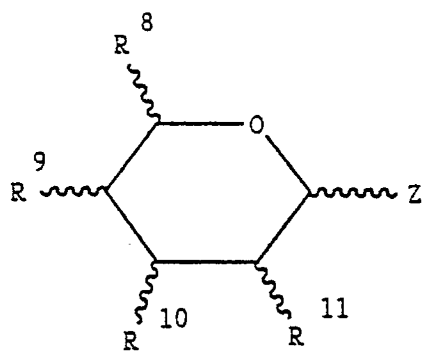

VI

worin

$R^8$

Methyl, Acyloxymethylen (C1-C8), Alkyloxymethylen (C1-C8), Benzyloxymethylen, Allyloxymethylen, Carboxymethyl, Carboxybenzyl oder Carboxyalkyl,

$R^9$, $R^{10}$ und $R^{11}$

unabhängig voneinander Wasserstoff, Acyloxy (C1-C8), Benzoyloxy oder substituiertes Benzoyloxy wie para-Nitrobenzoyloxy, Alkyloxy (C1-C8), Allyloxy, Benzyloxy, substituiertes Benzyloxy NH-Acyl oder NH-(9-Fluorenylmethoxycarbonyl) und

Z

eine Austrittsgruppe wie ein Halogenatom, eine Hydroxygruppe, eine Tri-C1-C4-alkylsilyloxy-Gruppe, eine über ein Sauerstoffatom gebundene Acyl-Schutzgruppe, oder ein über Sauerstoffatom gebundenes Trichloracetimidat

darstellen,

in Gegenwart eines Promotors und gegebenenfalls eines Säurefängers und/oder Trockenmittels in einem Lösungsmittel bei -50° bis +80° C zu einem 14-O-Glycosyl-Anthracyclinderivat der Formel I, worin alle Reste $R^1$ bis $R^{11}$ ihre Bedeutung wie oben definiert beibehalten, umsetzt, und in diesen Verbindungen gegebenenfalls die vorhandenen Schutzgruppen oder einiger der Schutzgruppen hydrolytisch oder mit Hilfe eines geeigneten Katalysators abspaltet.